(19) Europäisches Patentamt
European Patent Office
Office européen des brevets

(11) EP 0 854 146 B1

(12) EUROPEAN PATENT SPECIFICATION

(45) Date of publication and mention of the grant of the patent:
**15.03.2000 Bulletin 2000/11**

(51) Int Cl.⁷: **C07D 513/06**, A61K 31/47
// (C07D513/06, 285:00, 221:00)

(21) Application number: **98300249.4**

(22) Date of filing: **14.01.1998**

(54) **Substituted 4-(6-fluoro-[1H]-indol-3-yl)-1,2,3,6-tetrahydropyridine for the treatment of CNS-disorders**

Substituierte 4-(6-fluor-[1H]-indol-3-yl)1,2,3,6-Tetrahydropyridin-Derivate für die Behandlung von ZNS-Störungen

4-(6-fluoro-[1H]-indole-3-yl)1,2,3,6-tetrahydropyridine substituées pour le traitement des troubles du SNC

(84) Designated Contracting States:
**AT BE CH DE DK ES FI FR GB GR IE IT LI LU NL PT SE**
Designated Extension States:
**AL LT LV MK RO SI**

(30) Priority: **17.01.1997 GB 9700895**

(43) Date of publication of application:
**22.07.1998 Bulletin 1998/30**

(73) Proprietor: **ELI LILLY AND COMPANY LIMITED**
**Basingstoke, Hants RG21 6XA (GB)**

(72) Inventor: **Fairhurst, John,**
**c/o Eli Lilly and Company Ltd.**
**Windlesham, Surrey GU20 6PH (GB)**

(74) Representative: **Hudson, Christopher Mark et al**
**Eli Lilly and Company Limited,**
**Lilly Research Centre,**
**Erl Wood Manor**
**Windlesham Surrey GU20 6PH (GB)**

(56) References cited:
**EP-A- 0 433 149**
**EP-A- 0 465 398**
**EP-A- 0 722 941**
**DE-A- 19 500 689**
**FR-A- 2 675 801**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to pharmaceutical compounds and their use in the treatment of disorders of the central nervous system.

It is known that changes occur at certain neuronal serotonin (5-HT) receptors as, for instance, the 5-HT2A receptor, in central nervous system disorders such as, for example, depression. One aim of research for better drugs is to provide compounds that bind to specific receptors, such as the 5-HT2A receptor, and that also have beneficial activity at other receptors to give a desired profile of activity without, or while minimising, unwanted side effects. For example, it can be desirable for a drug to combine affinity for the serotonin receptor and also to inhibit serotonin reuptake, whilst showing low binding at the $\alpha_1$-adrenergic and dopamine D2 receptors. A high affinity for the $\alpha_1$ and D2 receptors is associated with unwanted cardiovascular and motor side effects.

Certain compounds having serotonin antagonist properties are described in EP-A 0 433 149.

The present invention provides compounds of the formula:

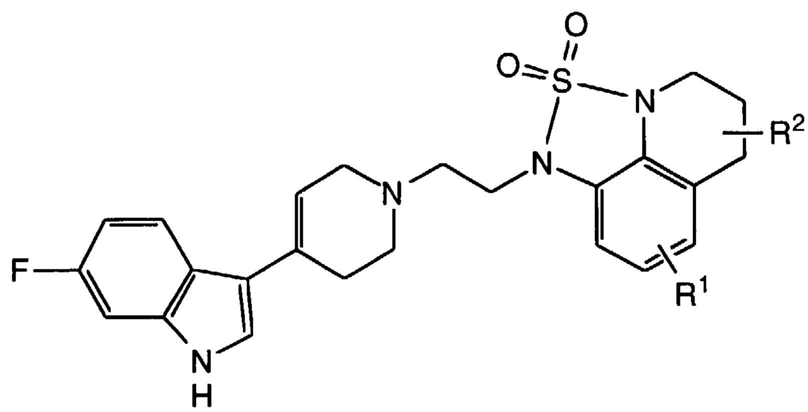

**(I)**

in which $R^1$ is hydrogen, $C_{1-4}$ alkyl, $C_{1-4}$ alkoxy or halo, and $R^2$ is hydrogen, $C_{1-4}$ alkyl or $C_{1-4}$ alkoxy; and salts thereof.

The compounds of the invention and their pharmaceutically acceptable salts are indicated for use in the treatment of disorders of the central nervous system. They are active in *in vitro* and *in vivo* tests that indicate serotonergic modulation and, in particular, binding activity at the 5-HT2A receptor, as described below. In this regard, the compounds of the invention are surprisingly superior to the prior art. They also strongly inhibit serotonin reuptake and have very low affinity at $\alpha$1 and D2 receptors.

In the above formula (I), a $C_{1-4}$ alkyl group can be branched or unbranched and, for example, includes methyl, ethyl, propyl, isopropyl, butyl, isobutyl and t-butyl, and is preferably methyl or ethyl, and especially methyl. A $C_{1-4}$ alkoxy group is one such alkyl group linked to the ring through an oxygen atom, and is preferably methoxy or ethoxy, and especially methoxy. A halo group is preferably chloro or fluoro, and especially fluoro.

A preferred group of compounds is one of formula (I) above, in which both $R^1$ and $R^2$ are hydrogen, or either $R^1$ or $R^2$ is hydrogen; and salts thereof. Preferably $R^1$ is hydrogen, methyl, methoxy or fluoro, and $R^2$ is preferably hydrogen or methyl.

A most preferred compound is 1-{2-[4-(6-fluoro-1H-indol-3-yl)-1,2,3,6-tetrahydro-1-pyridinyl]-1-ethyl}-5,6-dihydro-1H,4H-1,2,5-thiadiazolo[4,3,2-ij]quinoline-2,2-dioxide, and its salts, having the structure of formula (I) above, in which both $R^1$ and $R^2$ are hydrogen.

As indicated above, it is, of course, possible to prepare salts of the compounds of the invention and such salts are included in the invention. Acid addition salts are preferably the pharmaceutically acceptable, non-toxic addition salts with suitable acids, such as those with inorganic acids, for example hydrochloric, hydrobromic, nitric, sulphuric or phosphoric acids, or with organic acids, such as organic carboxylic acids, for example glycollic, maleic, hydroxymaleic, fumaric, malic, tartaric, citric, salicyclic, o-acetoxybenzoic, or organic sulphonic, 2-hydroxyethane sulphonic, toluene-p-sulphonic, naphthalene-2-sulphonic or bisethane sulphonic acids. The phosphate is a most preferred salt.

In addition to the pharmaceutically acceptable salts, other salts are included in the invention. They may serve as intermediates in the purification of compounds or in the preparation of compounds or in the preparation of other, for example pharmaceutically acceptable acid addition salts, or are useful for identification, characterisation or purification.

It will be appreciated that the compounds of the invention can contain one or more asymmetric carbon atoms which gives rise to isomers. The compounds are normally prepared as racemic mixtures, but individual isomers can be isolated by conventional techniques if so desired. Such racemic mixtures and individual optical isomers form part of the present invention, the compounds being employed as racemates or in enantiomerically pure form.

The invention includes a process for producing the compounds of the invention. The compounds can be prepared, for example, by the reaction of an indolyl piperidinyl compound of the formula:

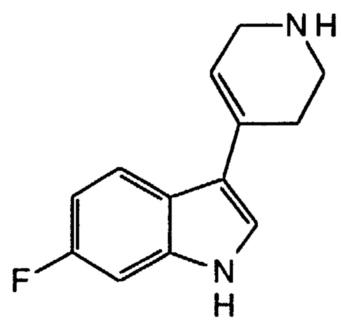

**(II)**

with a thiadiazolo quinoline dioxide of the formula:

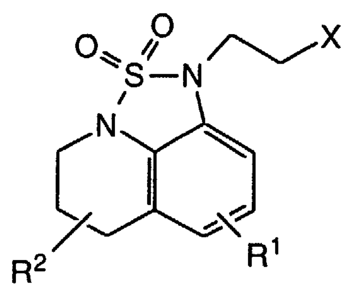

**(III)**

where X is a leaving group such as halo, especially chloro. The reaction is preferably carried out in a polar solvent such as, for example, acetonitrile, or water, in the presence of a base such as, for example, sodium carbonate, and at a temperature of from 50° C. to 150° C.

The compound of formula (II) can be prepared by methods known in the art, for example, by reacting 4-piperidone with 6-fluoroindole in the presence of a base such as potassium hydroxide, and employing as solvent an alcohol such as methanol. Similarly, methods for preparing the compounds of formula (III) are known in the art as, for example, by reacting the thiadiazolo quinoline dioxide in salt form with 1,2 dihaloethane, in the presence of sodium hydride and a suitable solvent such as dimethyl formamide.

As mentioned above, the compounds of the invention are active at the serotonin, 5-HT2A, receptor. Their binding activity has been demonstrated in the test described by Nelson, D. L. et al., J. Pharmacol. Exp. Ther., 265, 1272-1279, in which the affinity of the compound for the human 2A receptor is measured by its ability to displace the ligand [$^3$H] ketanserin. In this test, the compounds of the invention in the following Examples had mean Ki's of less than 6 nM. They are at least four times more active in this test than the closest structurally related compound specifically disclosed in EP-A 0 433 149, namely, 1-{3-[4-(5-fluoro-1H-indol-3-yl)-1,2,3,6-tetrahydro-1-pyridinyl]-1-propyl}-5,6-dihydro-1H,4H-1,2,5-thiadiazolo[4,3,2-ij]quinoline-2,2-dioxide. Indeed, the most active compound of the invention, the compound in which both $R^1$ and $R^2$ are hydrogen, is approximately twenty-eight times more active.

The affinity of the compounds of the invention for the 5-HT2A receptor and their superiority was confirmed in

a guinea pig model by a modified version of the test described by Skingle, M. et al., J. Psychopharmacol. 8, 14-21, in which the effect of the compound on 2,5-dimethoxy-4-iodo-amphetamine(DOI)-induced hyperthermia is observed.

Furthermore, the compounds of the invention are serotonin reuptake inhibitors, and possess excellent activity as, for example, in the test described by Carroll et al., J. Med. Chem. (1993), 36, 2886-2890, in which the intrinsic activity of the compound to competitively inhibit the binding of selective serotonin reuptake inhibitors to the serotonin transporter is measured. These results were also confirmed by *in vivo* tests in which the effect of the compound on a behavioural syndrome in mice dosed with 5-HTP and a monoamine oxidase inhibitor (MAOI) such as pargyline, is measured, see Christensen, A. V., et al., Eur. J. Pharmacol. 41, 153-162 (1977).

As stated above, the compounds of the invention have an excellent receptor binding profile which not only combines exceptionally high 2A binding and excellent reuptake inhibition, but also has the required low affinity for $\alpha 1$ and D2 receptors. Measurement of the former is described in Greengrass P. et al., Eur. J. Pharmacol. 55: 323-326, and of the latter in Hall H. et al., Prog. Neuro-Psycopharmacol. Biol. Psychiat. 12: 559-568. This characteristic profile of activity distinguishes the compounds of the invention from the structurally related compound of the prior art, referred to above. Other prior art compounds such as those described in J. Med. Chem. 1991, 34, 2477-2483, and J. Med. Chem. 1993, 36, 1194-1202, have further structural differences or lack efficacy *in vivo.*

In view of the selective affinity of the compounds of the invention for the serotonin receptors, they are indicated for use in treating a variety of conditions such as depression, bipolar disorder, anxiety, obesity, eating disorders such as anorexia and bulimia, alcoholism, pain, hypertension, ageing, memory loss, sexual dysfunction, psychotic disorders, schizophrenia, gastrointestinal disorders, headache, cardiovascular disorders, smoking cessation, epilepsy, drug abuse and addiction, emesis, Alzheimer's disease and sleep disorders. The compounds of the invention are principally intended for the treatment of depression or anxiety, or disorders with depressive or anxiety symptoms.

The compounds of the invention are effective over a wide dosage range, the actual dose administered being dependent on such factors as the particular compound being used, the condition being treated and the type and size of animal being treated. However, the dosage required will normally fall within the range of 0.001 to 20, such as 0.01 to 20 mg/kg per day, for example in the treatment of adult humans, dosages of from 0.5 to 100 or 200 mg per day may be used.

The compounds of the invention will normally be administered orally or by injection and, for this purpose, the compounds will usually be utilised in the form of a pharmaceutical composition. Such compositions are prepared in a manner well known in the pharmaceutical art and comprise at least one active compound.

Accordingly the invention includes a pharmaceutical composition comprising as active ingredient a compound of formula (I) or a pharmaceutically acceptable salt thereof, associated with a pharmaceutically acceptable diluent or carrier. In making the compositions of the invention, the active ingredient will usually be mixed with a carrier, or diluted by a carrier, or enclosed within a carrier which may be in the form of a capsule, sachet, paper or other container. More than one active ingredient or excipient may, of course, be employed. The excipient may be a solid, semi-solid or liquid material which acts as a vehicle, excipient or medium for the active ingredient. Some examples of suitable excipients are lactose, dextrose, sucrose, sorbitol, mannitol, starches, gum acacia, calcium phosphate, alginates, tragacanth, gelatin, syrup, methyl cellulose, methyl- and propylhydroxybenzoate, talc, magnesium stearate or oil. The compositions of the invention may, if desired, be formulated so as to provide quick, sustained or delayed release of the active ingredient after administration to the patient.

Depending on the route of administration, the foregoing compositions may be formulated as tablets, capsules or suspensions for oral use and injection solutions or suspensions for parenteral use or as suppositories. Preferably the compositions are formulated in a dosage unit form, each dosage containing from 0.5 to 100 mg, more usually 1 to 100 mg, of the active ingredient.

The following Examples illustrate the preparation of compounds of the invention:

EXAMPLE 1

1-Dimethylamino-2-(4-fluoro-2-nitro)phenylethene

A mixture of 4-fluoro-2-nitrotoluene (50 g, 0.32 mol), dimethylformamide dimethylacetal (76.77 g) and dimethylformamide (910 ml) were heated at reflux under nitrogen with stirring for 7 hours, cooled, allowed to stand for 16 hours, poured into ice-water (2000 ml), stirred for 15 minutes, the resultant precipitate was isolated by filtration, washed with water (500 ml) and dried to give a red solid.

6-Fluoroindole

A 40 litre Cook hydrogenator was charged under a nitrogen atmosphere with 10% palladium on charcoal (9 g) suspended in toluene (400 ml). To this suspension was added 1-dimethylamino-2-(4-fluoro-2-nitro)phenylethene

(137.2 g, 0.653 mol) in toluene (1400 ml) and the mixture hydrogenated at 80 psi for 3.5 hours. The suspension was then filtered through a celite pad, which was washed through with toluene (2 x 200 ml) and the filtrate and washings evaporated under reduced pressure to give a brown oil which crystallised on standing to a yellow brown solid 93.65 g. This solid was dissolved in ethyl acetate-hexane (7:3) and filtered through a pad of flash silica. The required fractions were collected and evaporated under reduced pressure to give a pale brown solid.

4- (6-fluoro-[1H]-indol-3-yl)-1,2,3,6-tetrahydropyridine

Powdered potassium hydroxide (144.4 g) was added carefully to a mechanically stirred mixture of 6-fluoroindole (49.23 g, 0.364 mol) and piperidone hydrochloride monohydrate (111.93 g, 0.728 mol) in methanol (1500 ml). The mixture was then heated at reflux under nitrogen for 18 hours and then more potassium hydroxide (40 g) was added and the reaction mixture heated under reflux for a further 4 hours. The reaction mixture was allowed to cool to room temperature, poured onto ice-water (3000 ml) and stirred for 1 hour. The precipitated solid was isolated by filtration and dried at 50° C. *in vacuo* to give a solid product.

8-Amino-1,2,3,4-tetrahydroquinoline

8-Nitroquinoline (25 g, 0.143 mol) was dissolved in acetic acid (300 mL) and catalytically hydrogenated over $PtO_2$ (0.75 g) at 65 psi overnight at room temperature. The catalyst was removed by filtration through a celite pad and the solvent was removed by evaporation. The residue was dissolved in $CH_2Cl_2$ and saturated aqueous sodium hydrogen carbonate added. The organics were extracted with $CH_2Cl_2$ and the extracts were washed with water (3 x 100 mL) and brine (3 x 100 mL). The extracts were then dried over anhydrous magnesium sulphate, filtered and evaporated to leave the crude product as a brown oil. This was immediately dissolved in $CH_2Cl_2$ and filtered through a short pad of silica eluting with ethyl acetate to leave the title compound.

5,6-Dihydro-1H-4H-1,2,5-thiadiazolo-[4,3,2-ij]-quinoline-2,2-dioxide, potassium salt

The 8-aminotetrahydroquinoline (6.60 g, 0.045 mol) in dry diglyme (25 mL) was added to a stirred solution of sulfamide (4.93 g, 0.051 mol) in dry diglyme (40 mL) at 155-160° C. The reaction was then heated for 2 hours. After this time, the reaction was cooled to room temperature, water was added and the mixture acidified with 1N hydrochloric acid. The solution was extracted with t-butyl ether (4 x 100 mL). The extracts were washed with water (2 x 100 mL) and then charcoal was added and filtered off to leave a clear red solution. 8N potassium hydroxide was added dropwise to the solution precipitating a white solid. This was filtered off and washed with ether to give the required product.

2-Chloroethyl-5,6-dihydro-1H,4H-1,2,5-thiadiazolo[4,3,2-ij]quinoline-2,2-dioxide

5,6-Dihydro-1H-4H-1,2,5-thiadiazolo-[4,3,2-ij]-quinoline-2,2-dioxide (1.92 g, 0.009 mol) was dissolved in dry DMF (80 mL) and sodium hydride (0.48 g, 0.010 mol) was added portionwise under nitrogen to the mixture at room temperature. Bromochloroethane (1.44 g, 0.010 mol) was added in one portion and the solution stirred overnight at ambient temperature. The reaction was quenched with water, extracted with ethyl acetate, the combined extracts washed with brine, dried over anhydrous magnesium sulphate, filtered and evaporated under reduced pressure to give a brown oil. The material was purified by chromatography on flash silica eluting with 20% ethyl acetate/hexane. The product was obtained as a clear oil which crystallised on standing.

1-{2-[4-(6-Fluoro- [1H]-indol-3-yl)-1,2,3,6-tetrahydro-1-pyridinyl]-1-ethyl}-5,6-dihydro-1H,4H-1,2,5 -thiadiazolo [4,3,2-ij]quinoline-2,2-dioxide

2-Chloroethyl-5,6-dihydro-1H,4H-1,2,5-thiadiazolo[4,3,2-ij]quinoline-2,2-dioxide (3.75 g, 0.0137 mol) and 4-(6-fluoro-[1H]-indol-3-yl)1,2,3,6-tetrahydropyridine (2.97 g, 0.0137 mol) in water (120 mL) containing sodium carbonate (7.26 g, 0.0085 mol) with a catalytic amount of sodium iodide (0.08 g) were heated at reflux for 18 hours. After cooling, the material was extracted with dichloromethane, the extracts dried with anhydrous magnesium sulphate, filtered and evaporated to leave an orange foam. This material was purified by chromatography eluting with dichloromethane, followed by 5% methanol/dichloromethane giving the product as a pale yellow solid. The product was recrystallised from ethyl acetate, m.p. 183-185° C.

The free base was dissolved in methanol, orthophosphoric acid was added and the white precipitate filtered and dried *in vacuo* to yield the phosphate salt, m.p. 156-158° C.

EXAMPLE 2

Preparation of intermediates

6-Fluoro-8-nitroquinoline

Sulfuric acid (75% w/w) (116 g) was added to a mixture of sodium 3-nitrobenzene sulfonate (46.4 g, 0.206 mol), 2-nitro-4-fluoroaniline (25 g, 0.156 mol) and glycerol (29 g, 0.315 mol) and slowly heated to 130-135° C. and maintained at this temperature for 8 hours. It was cooled and allowed to stand for 11 hours, diluted with water (100 ml), basified with ammonia (d = 0.88 g/ml) and the precipitated solid filtered. The filtrate was washed with sodium hydroxide (2M, 2 x 50 ml) and water (3 x 50 ml), air dried and then dried *in vacuo* at 60° C. for 4 hours to give 6-fluoro-8-nitroquinoline as a solid.

8-Amino-6-fluoro-1,2,3,4-tetrahydroquinoline

Platinum oxide (0.475 g) was added to a solution of 6-fluoro-8-nitroquinoline (14.0 g, 72.9 mmol) in acetic acid (195 ml) and hydrogenated at 60 psi for 24 hours. The acetic acid solution was filtered through celite to remove the catalyst and then evaporated *in vacuo* and the residue treated with toluene (150 ml). The toluene was removed *in vacuo* and more toluene was added. The resultant solution was filtered and then evaporated *in vacuo.* The crude product, a dark liquid, was used immediately in the next step.

8-Fluoro-5,6-dihydro-1H,4H-1,2,5-thiadiazolo-[4,3,2-i,j]-quinoline-2,2-dioxide

Crude 8-amino-6-fluoro-1,2,3,4-tetrahydroquinoline (10.34 g, 62.29 mmol) in dry pyridine (60 ml) was added to hot sulfamide (7.026 g, 74.75 mmol) in dry pyridine (60 ml) and the mixture heated and stirred under reflux for 4 hours. It was cooled and allowed to stand over-night. The pyridine was evaporated *in vacuo* to give a black residue which was partitioned between ethyl acetate (300 ml) and 2M hydrochloric acid (195 ml). The mixture was shaken, separated and washed with more 2M hydrochloric acid (100 ml), water (100 ml) and then separated again. The solid was dried with magnesium sulfate, the magnesium sulfate removed by filtration, and the filtrate treated with charcoal. The charcoal was then removed by filtration, the filtrate evaporated and then evaporated *in vacuo,* to give a slightly pink solid, m.p. 174-176° C.

3-Methyl-8-nitroquinoline

Sulfuric acid (34 ml, 63.2 g, 645.2 mmol) in water (12 ml) was added to a mixture of arsenic pentoxide (22.26 g, 96.8 mmol) and 2-nitroaniline (22.28 g, 161.3 mmol) and the solids dissolved. The resultant solution was then heated to 100° C. and 2-methyl-2-propene-1,1-diol diacetate (50 g, 290.3 mmol) was added, causing an exothermic reaction which was controlled so as not to exceed 130° C. After the addition of the 2-methyl-2-propene-1,1-diol diacetate, the mixture was heated and stirred at 130° C. for 2 hours. The reaction mixture was then cooled and poured onto ice-water. The resultant mixture was basified with aqueous sodium hydroxide solution (50%) and toluene was then added and the mixture heated to 90° C. for 1 hour. The toluene layer was then decanted off and replaced with more toluene (400 ml). The mixture was then heated and stirred over-night and the toluene decanted. More toluene (400 ml) was then added and the mixture heated and stirred for 2 hours before decanting and combining with the two previous toluene extracts. The combined toluene extracts were dried ($MgSO_4$), filtered and the filtrate evaporated *in vacuo* to give a dark brown solid. This solid was triturated with diethyl ether, the solid isolated by filtration, pulverised, washed with ether and dried *in vacuo* at 50° C for 2 hours to give the solid product.

8-Amino-3-methyl-1,2,3,4-tetrahydroquinoline

Platinum oxide (0.375 g) was added to a solution of 3-methyl-8-nitroquinoline (12.47 g, 66.3 mmol) in acetic acid (150 ml) and hydrogenated at 60 psi for 24 hours.

The acetic acid solution was filtered through celite to remove the catalyst and then evaporated *in vacuo* and the residue treated with toluene (150 ml). The toluene was removed *in vacuo.* More toluene was added and the resultant solution filtered and then evaporated *in vacuo.* The crude product was then dissolved in ethyl acetate and purified by chromatography on a pad of silica. The ethyl acetate fractions containing product were collected and combined, then washed with aqueous sodium hydrogen carbonate solution (75 ml), dried ($MgSO_4$) and filtered. The filtrate was evaporated *in vacuo* to give a dark brown oil.

5-Methyl-5,6-dihydro-1H,4H-1,2,5-thiadiazolo-[4,3,2-i,j]-quinoline-2,2-dioxide potassium salt

8-Amino-3-methyl-1,2,3,4-tetrahydroquinoline (7.56 g, 46.66 mmol) in dry pyridine (50 ml) was added to hot sulfamide (5.38 g, 56 mmol) in dry pyridine (50 ml) and the mixture heated and stirred mechanically under reflux for 2 hours. The pyridine was evaporated *in vacuo* to give a black residue which was partitioned between ethyl acetate and 2.5M hydrochloric acid. The acid layer was separated and the organic layer washed with brine. The aqueous acid layer was then extracted with ethyl acetate (3x) combined with the original ethyl acetate layer, treated with charcoal, dried with magnesium sulfate, filtered and the filtrate evaporated *in vacuo* to give an orange solid. This solid was dissolved in the minimum of tert-butyl methyl ether and the resultant solution treated with 8M potassium hydroxide solution. The precipitated cream coloured solid was collected by filtration and dried *in vacuo* at 50° C. to give the potassium salt as a solid, m.p. >250° C.
The following compounds of the invention were prepared as in Example 1, using intermediates prepared in the manner described above.
R,S 1-(2-(4-(6-Fluoroindol-3-yl)-1,2,3,6-tetrahydropyridin-1-yl)ethyl)-5,6-dihydro-4-methyl-1H,4H-1,2,5-thiadiazolo-[4,3,2-i,j]-quinoline-2,2-dioxide, m.p. 196-197° C.
R,S 1-(2-(4-(6-Fluoroindol-3-yl)-1,2,3,6-tetrahydropyridin-1-yl)ethyl)-5,6-dihydro-5-methyl-1H,4H-1,2,5-thiadiazolo-[4,3,2-i,j]-quinoline-2,2-dioxide, m.p. 197-200° C.
R,S 1-(2-(4-(6-Fluoroindol-3-yl)-1,2,3,6-tetrahydropyridin-1-yl)ethyl)-5,6-dihydro-6-methyl-1H,4H-1,2,5-thiadiazolo-[4,3,2-i,j]-quinoline-2,2-dioxide, m.p. 156-158° C.
1-(2-(4-(6-Fluoroindol-3-yl)-1,2,3,6-tetrahydropyridin-1-yl)ethyl)-5,6-dihydro-7-methyl-1H,4H-1,2,5-thiadiazolo-[4,3,2-i,j]-quinoline-2,2-dioxide, m.p. 187.8-189.7° C.
1-(2-(4-(6-Fluoroindol-3-yl)-1,2,3,6-tetrahydropyridin-1-yl)ethyl)-5,6-dihydro-8-methyl-1H,4H-1,2,5-thiadiazolo-[4,3,2-i,j]-quinoline-2,2-dioxide, m.p. 169.4-174° C.
1-(2-(4-(6-Fluoroindol-3-yl)-1,2,3,6-tetrahydropyridin-1-yl)ethyl)-5,6-dihydro-9-methyl-1H,4H-1,2,5-thiadiazolo-[4,3,2-i,j]-quinoline-2,2-dioxide, m.p. 189.2-190.3° C.
7-Fluoro-1-(2-(4-(6-fluoroindol-3-yl)-1,2,3,6-tetrahydropyridin-1-yl)ethyl)-5,6-dihydro-1H,4H-1,2,5-thiadiazolo-[4,3,2-i,j]-quinoline-2,2-dioxide, 178-178.4° C.
8-Fluoro-1-(2-(4-(6-fluoroindol-3-yl)-1,2,3,6-tetrahydropyridin-1-yl)ethyl)-5,6-dihydro-1H,4H-1,2,5-thiadiazolo-[4,3,2-i,j]-quinoline-2,2-dioxide, m.p. 173-175° C.
9-Fluoro-1-(2-(4-(6-fluoroindol-3-yl)-1,2,3,6-tetrahydropyridin-1-yl)ethyl)-5,6-dihydro-1H,4H-1,2,5-thiadiazolo-[4,3,2-i,j]-quinoline-2,2-dioxide, m.p. 188-190° C.
1-(2-(4-(6-Fluoroindol-3-yl)-1,2,3,6-tetrahydropyridin-1-yl)ethyl)-5,6-dihydro-9-ethyl-1H,4H-1,2,5-thiadiazolo-[4,3,2-i,j]-quinoline-2,2-dioxide, m.p. 166.4-168° C.
1-(2-(4-(6-Fluoroindol-3-yl)-1,2,3,6-tetrahydropyridin-1-yl)ethyl)-5,6-dihydro-9-methoxy-1H,4H-1,2,5-thiadiazolo-[4,3,2-i,j]-quinoline-2,2-dioxide, 187-189° C.
The following Examples illustrate typical formulations containing a compound of the invention.

EXAMPLE 3

Tablets each containing 10 mg of active ingredient are made up as follows:

| | |
|---|---|
| Active ingredient | 10 mg |
| Starch | 160 mg |
| Microcrystalline cellulose | 100 mg |
| Polyvinylpyrrolidone (as 10% solution in water) | 13 mg |
| Sodium carboxymethyl starch | 14 mg |
| Magnesium stearate | 3 mg |
| Total | 300 mg |

The active ingredient, starch and cellulose are mixed thoroughly. The solution of polyvinylpyrrolidone is mixed with the resultant powders and passed through a sieve. The granules so produced are dried and re-passed through a sieve. The sodium carboxymethyl starch and magnesium stearate are then added to the granules which, after mixing, are compressed on a tablet machine to yield tablets each weighing 300 mg.

EXAMPLE 4

Capsules each containing 20 mg of active ingredient are made as follows:

| | |
|---|---|
| Active ingredient | 20 mg |
| Dried starch | 178 mg |
| Magnesium stearate | 2 mg |
| Total | 200 mg |

The active ingredient, starch and magnesium stearate are passed through a sieve and filled into hard gelatine capsules in 200 mg quantities.

EXAMPLE 5

Capsules each containing 20 mg of medicament are made as follows:

| | |
|---|---|
| Active ingredient | 20 mg |
| Lactose | 171 mg |
| Sodium lauryl sulphate | 2 mg |
| Sodium starch glycollate | 6 mg |
| Magnesium stearate | 1 mg |
| | 200 mg |

The active ingredient, lactose, sodium lauryl sulphate and sodium starch glycollate are mixed thoroughly. The blend is mixed with the magnesium stearate and filled into hard gelatine capsules in 200 mg quantities.

EXAMPLE 6

Tablets each containing 20 mg and medicaments are made as follows:

| | |
|---|---|
| Active ingredient | 20 mg |
| Lactose | 103 mg |
| Microcrystalline cellulose | 150 mg |
| Hydroxypropylmethylcellulose | 15 mg |
| Sodium starch glycollate | 9 mg |
| Magnesium stearate | 3 mg |
| | 300 mg |

The active ingredient, lactose, microcrystalline cellulose, sodium starch glycollate and hydroxypropylmethylcellulose are passed through a sieve and blended together. Water is added to the blended powders to form a damp mass. The damp mass is passed through a coarse screen, dried, then re-screened. The dried granules are mixed with the magnesium stearate and compressed into tablets of 300 mg weight.

**Claims**

**1.** A compound of the formula:

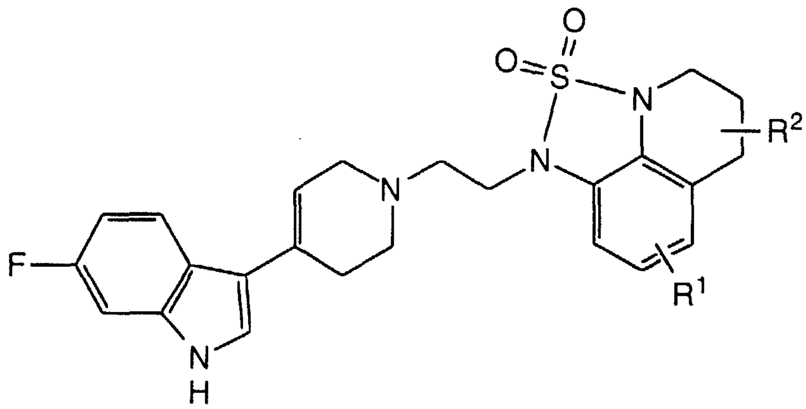

**(I)**

in which $R^1$ is hydrogen, $C_{1-4}$ alkyl, $C_{1-4}$ alkoxy or halo, and $R^2$ is hydrogen, $C_{1-4}$ alkyl or $C_{1-4}$ alkoxy; or a salt thereof.

**2.** A compound according to Claim 1 in which both $R^1$ and $R^2$ are hydrogen, or either $R^1$ or $R^2$ is hydrogen.

**3.** A compound according to either of Claims 1 and 2 in which $R^1$ is hydrogen, methyl, methoxy or fluoro, and $R^2$ is hydrogen or methyl.

**4.** 1-{2-[4-(6-Fluoro-1H-indol-3-yl)-1,2,3,6-tetrahydro-1-pyridinyl]-1-ethyl}-5,6-dihydro-1H,4H-1,2,5-thiadiazolo [4,3,2-ij]quinoline-2,2-dioxide, or a salt thereof.

**5.** 1-{2-[4-(6-Fluoro-1H-indol-3-yl)-1,2,3,6-tetrahydro-1-pyridinyl]-1-ethyl}-5,6-dihydro-1H,4H-1,2,5-thiadiazolo [4,3,2-ij]quinoline-2,2-dioxide.

**6.** 1-{2-[4-(6-Fluoro-1H-indol-3-yl)-1,2,3,6-tetrahydro-1-pyridinyl]-1-ethyl}-5,6-dihydro-1H,4H-1,2,5-thiadiazolo [4,3,2-ij]quinoline-2,2-dioxide phosphate salt.

**7.** A pharmaceutical formulation comprising a compound according to any of Claims 1 to 4 or a pharmaceutically acceptable salt thereof, or a compound according to either of Claims 5 and 6, associated with a pharmaceutically acceptable diluent or carrier therefor.

**8.** A compound according to any of Claims 1 to 4, or a pharmaceutically acceptable salt thereof, or a compound according to either of Claims 5 and 6, for use as a pharmaceutical.

**9.** A compound according to Claim 1 or a pharmaceutically acceptable salt thereof, for the treatment of depression or anxiety, having high binding activity at the 5-HT2A receptor.

**10.** The use of a compound according to any of Claims 1 to 4 or a pharmaceutically acceptable salt thereof, or a compound according to either of Claims 5 and 6, for the manufacture of a medicament for the treatment of a disorder of the central nervous system.

**11.** A process for producing a compound according to Claim 1, which comprises reacting a compound of the formula:

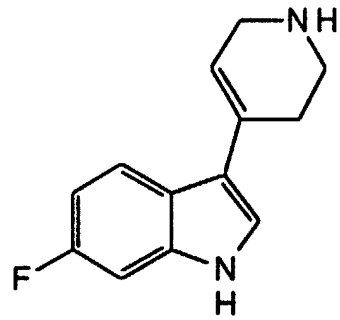

**(II)**

with a compound of the formula:

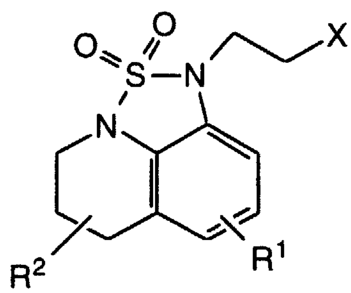

**(III)**

where X is a leaving group.

**12.** A compound according to claim 1 produced by a process according to Claim 11.

**13.** A process for producing a pharmaceutical formulation which comprises admixing a compound according to any of Claims 1 to 4 or a pharmaceutically acceptable salt thereof, or a compound according to either of Claims 5 and 6, with a pharmaceutically acceptable diluent or carrier therefor.

**14.** A dosage unit form containing from 0.5 to 100 mg of a compound according to Claim 1 or a pharmaceutically acceptable salt thereof, as active ingredient.

**Revendications**

**1.** Composé de formule

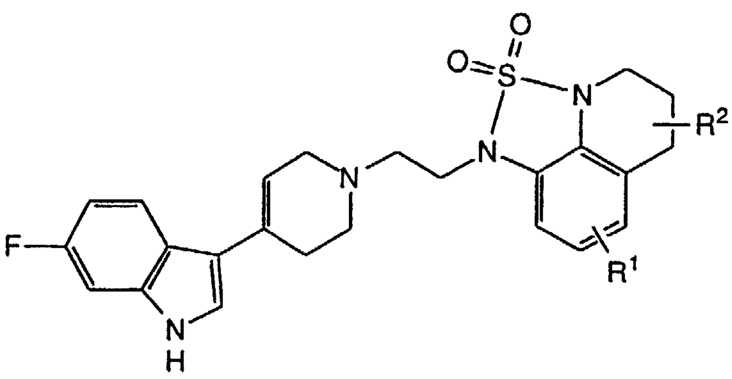

**(I)**

dans laquelle $R^1$ représente l'atome d'hydrogène, un groupe alkyle en $C_{1-4}$, un groupe alcoxy en $C_{1-4}$ ou un atome d'halogène, et $R^2$ représente l'atome d'hydrogène, un groupe alkyle en $C_{1-4}$ ou un groupe alcoxy en $C_{1-4}$ ; ou un sel de celui-ci.

**2.** Composé selon la revendication 1, dans lequel $R^1$ et $R^2$ sont tous les deux de l'hydrogène, ou dans lequel $R^1$ ou $R^2$ représentent de l'hydrogène.

**3.** Composé selon l'une quelconque des revendications 1 et 2, dans lequel $R^1$ représente de l'hydrogène, le groupe méthyle, le groupe méthoxy ou l'atome de fluor et $R^2$ représente de l'hydrogène ou le groupe méthyle.

**4.** 2,2-dioxyde de 1-{2-[4-(6-fluoro-1H-indol-3-yl)-1,2,3,6-tétrahydro-1-pyridinyl]-1-éthyl}-5,6-dihydro-1H,4H-1,2,5-thiadiazolo-[4,3,2-ij]-quinoléine ou un sel de celui-ci.

**5.** 2,2-dioxyde de 1-{2-[4-(6-fluoro-1H-indol-3-yl)-1,2,3,6-tétrahydro-1-pyridinyl]-1-éthyl}-5,6-dihydro-1H,4H-1,2,5-thiadiazolo-[4,3,2-ij]-quinoléine.

**6.** Sel phosphaté de 2,2-dioxyde de 1-{2-[4-(6-fluoro-1H-indol-3-yl)-1,2,3,6-tétrahydro-1-pyridinyl]-1-éthyl}-5,6-dihydro-1H,4H-1,2,5-thiadiazolo-[4,3,2-ij]-quinoléine.

**7.** Formulation pharmaceutique comprenant un composé selon l'une quelconque des revendications 1 à 4 ou un sel pharmaceutiquement acceptable de celui-ci, ou un composé selon l'une quelconque des revendications 5 et 6, associé à un diluant ou un support pharmaceutiquement acceptable pour celui-ci.

**8.** Composé selon l'une quelconque des revendications 1 à 4, ou un sel pharmaceutiquement acceptable de celui-ci, ou un composé selon l'une quelconque des revendications 5 et 6 destiné à être utilisé comme produit pharmaceutique.

**9.** Composé selon la revendication 1 ou un sel pharmaceutiquement acceptable de celui-ci pour le traitement de la dépression ou de l'anxiété, présentant une activité de fixation élevée sur le récepteur 5-HT2A.

**10.** Utilisation d'un composé selon l'une quelconque des revendications 1 à 4, ou d'un sel pharmaceutiquement acceptable de celui-ci, ou d'un composé selon l'une quelconque des revendications 5 et 6 pour la préparation d'un médicament pour le traitement d'un trouble du système nerveux central.

**11.** Procédé pour la préparation d'un composé selon la revendication 1, dans lequel on fait réagir un composé de formule :

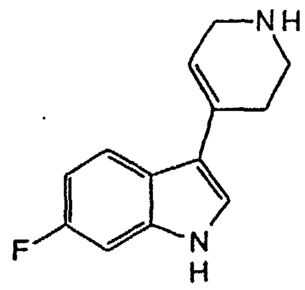

**(II)**

avec un composé de formule

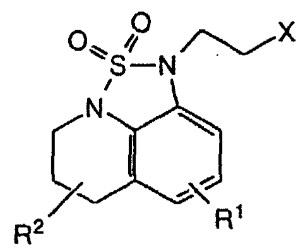

**(III)**

dans laquelle X est un groupe qui est éliminé.

**12.** Composé selon la revendication 1 préparé par un procédé selon la revendication 11.

**13.** Procédé pour la préparation d'une formulation pharmaceutique, comprenant le mélange d'un composé selon l'une quelconque des revendications 1 à 4 ou d'un sel pharmaceutiquement acceptable de celui-ci, ou d'un composé selon l'une quelconque des revendications 5 et 6, avec un diluant ou un support pharmaceutiquement acceptable pour celui-ci.

**14.** Forme de dosage unitaire contenant 0,5 à 100 mg d'un composé selon la revendication 1 ou d'un sel pharmaceutiquement acceptable de celui-ci, comme ingrédient actif.

**Patentansprüche**

**1.** Verbindung der Formel

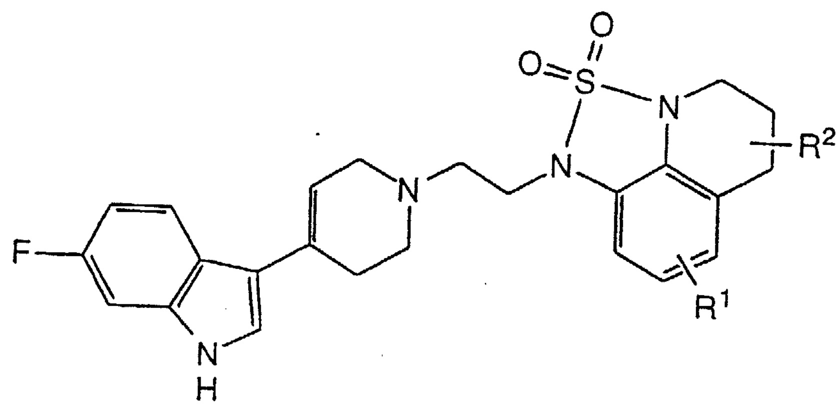

**(I)**

wobei $R^1$ Wasserstoff, $(C_1\text{-}C_4)$Alkyl, $(C_1\text{-}C_4)$Alkoxy oder Halo ist und $R^2$ Wasserstoff, $(C_1\text{-}C_4)$Alkyl oder $(C_1\text{-}C_4)$ Alkoxy ist; oder ein Salz davon.

**2.** Verbindung nach Anspruch 1, wobei sowohl $R^1$ als auch $R^2$ Wasserstoff sind, oder entweder $R^1$ oder $R^2$ Wasserstoff ist.

**3.** Verbindung nach einem der Ansprüche 1 und 2, wobei $R^1$ Wasserstoff; Methyl, Methoxy oder Fluoro ist, und $R^2$ Wasserstoff oder Methyl ist.

**4.** 1-(2-[4-(6-Fluoro-1H-indol-3-yl)-1,2,3,6-tetrahydro-1-pyridinyl]-1-ethyl)-5,6-dihydro-1H,4H-1,2,5-thiadiazolo-[4,3,2-ij]-chinolin-2,2-dioxid, oder ein Salz davon.

**5.** 1-{2-[4-(6-Fluoro-1H-indol-3-yl)-1,2,3,6-tetrahydro-1-pyridinyl]-1-ethyl}-5,6-dihydro-1H,4H-1,2,5-thiadiazolo-[4,3,2-ij]-chinolin-2,2-dioxid.

**6.** 1-{2-[4-(6-Fluoro-1H-indol-3-yl)-1,2,3,6-tetrahydro-1-pyridinyl]-1-ethyl}-5,6-dihydro-1H,4H-1,2,5-thiadiazolo-[4,3,2-ij]-chinolin-2,2-dioxidphosphatsalz.

**7.** Pharmazeutische Formulierung, umfassend eine Verbindung nach einem der Ansprüche 1 bis 4 oder ein pharmazeutisch verträgliches Salz davon, oder eine Verbindung entweder nach Anspruch 5 oder Anspruch 6, in Verbindung mit einem pharmazeutisch verträglichen Verdünner oder Träger dafür.

**8.** Verbindung nach einem der Ansprüche 1 bis 4, oder ein pharmazeutisch verträgliches Salz davon, oder eine Verbindung entweder nach Anspruch 5 oder Anspruch 6, zur Verwendung als Pharmazeutikum.

**9.** Verbindung nach Anspruch 1 oder ein pharmazeutisch verträgliches Salz davon für die Behandlung von Depression oder Angst, die eine hohe Bindungsaktivität am 5-HT2A-Rezeptor aufweisen.

**10.** Verwendung einer Verbindung nach den Ansprüchen 1 bis 4 oder eines pharmazeutisch verträglichen Salzes davon oder einer Verbindung nach entweder Anspruch 5 oder Anspruch 6 oder Herstellung eines Medikaments für die Behandlung einer Erkrankung des zentralen Nervensystems.

**11.** Verfahren zur Herstellung einer Verbindung nach Anspruch 1, welches Umsetzung einer Verbindung der Formel

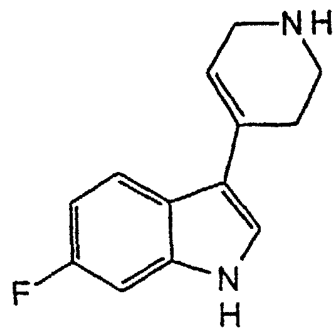

(II)

mit einer Verbindung der Formel:

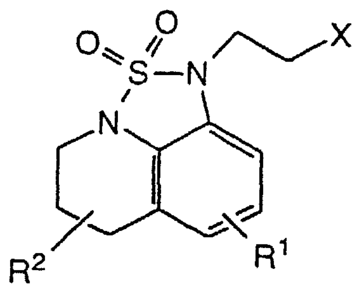

(III)

umfaßt, wobei X eine Abgangsgruppe ist.

12. Verbindung nach Anspruch 1, hergestellt durch ein Verfahren nach Anspruch 11.

13. Verfahren zur Herstellung einer pharmazeutischen Formulierung, welches Mischen einer Verbindung nach einem der Ansprüche 1 bis 4 oder eines pharmazeutisch verträglichen Salzes davon oder einer Verbindung entweder nach Anspruch 5 oder Anspruch 6 mit einem pharmazeutisch verträglichen Verdünner oder Träger dafür umfaßt.

14. Dosierungseinheitsform, enthaltend 0,5 bis 100 mg einer Verbindung nach Anspruch 1 oder eines pharmazeutisch verträglichen Salzes davon als aktiven Bestandteil.